# NEW EUROPEAN PATENT SPECIFICATION

(11) **EP 0 455 748 B2**
(45) Date of publication and mention of the opposition decision: **15.01.2003**
(45) Mention of the grant of the patent: 22.11.1995
(21) Application number: 90904062.8
(22) Date of filing: 03.01.1990
(51) Int. Cl.: C07C 19/08, C07C 17/00, C07C 17/20

(54) **MANUFACTURE OF 1,1,1,2-TETRAFLUOROETHANE**
VERFAHREN ZUR HERSTELLUNG VON 1,1,1,2-TETRAFLUOROÄTHAN
FABRICATION DE 1,1,1,2-TETRAFLUOROETHANE

(30) Priority: 03.02.1989 US 305697
(43) Date of publication of application: 13.11.1991
(73) Proprietor: E.I. DU PONT DE NEMOURS AND COMPANY, Wilmington Delaware 19898 (US)
(72) Inventor: MANZER, Leo, Ernest, Wilmington, DE 19803 (US)
(74) Representative: Beacham, Annabel Rose
(86) International application number: US9000011
(87) International publication number: WO90008755

(56) References cited:
- EP-A- 0 282 005
- EP-A- 0 328 127
- EP-A- 0 331 991
- GB-A- 2 030 981
- GB-A- 2 030 981
- US-A- 2 744 147
- US-A- 2 744 148
- US-A- 2 885 427
- US-A- 3 752 850
- US-A- 4 129 603
- US-A- 4 158 675
- US-A- 4 792 643

## Description

This invention relates to an improved catalytic process for the preparation of 1,1,1,2-tetrafluoroethane (HFC-134a) by the reaction of trichloroethylene with HF, whereby the reaction is conducted in a single reaction zone while recycling 2-chloro-1,1,1-trifluoroethane (HCFC-133a) with trichloroethylene to the reaction zone.

### BACKGROUND OF THE INVENTION

U.S. 2,637,747 discloses the reaction of trichloroethylene and HF over an antimony pentachloride catalyst to obtain a 32% yield of HCFC-133a, no HFC-134a, and some 1,2-dichloro-1,1-difluoroethylene.

U.S. 2,744,147 discloses an alumina catalyst, which may be promoted with a metal (cobalt, nickel, and chromium), and a process using the catalyst in a fluidized bed for fluorinating haloalkanes at a temperature between 180° to 425°C. CF₃CH₂Cl (HCFC-133a) is positively excluded from the list of halocarbons taught to be fluorinated by the invention process (Col. 1, lines 43-53, 54-65).

U.S. 2,744,148 discloses an alumina catalyst which may be promoted with a metal (chromium, cobalt, nickel, copper, and palladium) and a process for fluorinating haloalkanes to highly fluorinated products. A process is disclosed which activates the catalyst and converts at lease part of the alumina to basic aluminum fluorides. CF₃CH₂Cl (HCFC-133a) is again positively excluded from the list of halocarbons taught to be fluorinated by the invention process (Col. 2, lines 23-33, 34-46).

U.S. 2,885,427 claims a process for forming a 1,1,1-trifluoro-2-haloethane by the reaction of HF and trichloroethylene over a basic chromium fluoride catalyst. Example 1 shows that this reaction affords the following products with the selectivities shown; HCFC-133a (94.2%), HFC-134a (3.6%), 1,2-dichloro-1-fluoroethylene (2.0%), and pentafluoroethane (0.2%); the conversion of trichloroethylene is 92.3%.

U.S. 3,003,003 claims a process for the manufacture of HCFC-133a by reacting trichloroethylene and HF over an antimony fluorochloride catalyst. In Example 2 a 67% yield of HCFC-133a is reported for this reaction.

GB 1,000,485 claims a process for the preparation of fluorinated organic compounds by passing a halo-olefin and HF over a catalyst consisting essentially of activated alumina which is partially fluorinated. The alumina catalyst may also contain polyvalent metals chosen from chromium, cobalt, nickel, and manganese. The patent discloses that the use of AlF₃ as a catalyst is not always satisfactory partly because it "leads to several reaction by-products which may be present in large quantities" (page 2, lines 20-24). In Example 1, trichloroethylene is reacted with HF over a fluorinated alumina containing chromium and cobalt to yield HCFC-133a with a 94.1% selectivity, fluorinated olefins with a 2.7% selectivity and no HFC-134a.

U.S. 3,752,850 discloses a process for the manufacture of HCFC-133a by reacting trichloroethylene and HF in the presence of a catalyst, the composition of which can vary between CrF_{1.5}O_{1.5} and CrF₂O, or a catalyst, the composition of which can vary between CrFO₂ and CrF₂O. In Example 8 it is shown that the process of the invention affords HCFC-133a in 94.8% yield and no HCFC-134a.

U.S. 4,129,603 discloses a process for the manufacture of HFC-134a which comprises reacting in the vapor phase at elevated temperature a haloethane of formula CX₃CH₂Y, wherein X is Br, Cl, or F and Y is Cl, with HF in the presence of a catalyst which is chromium oxide or which is at least in part basic chromium fluoride and wherein the HFC-134a product containing 1-chloro-2,2-difluoroethylene, which is an impurity, is removed by intimate contact with a metal permanganate in a liquid medium.

U.S. 4,158,675 discloses a process for the manufacture of HFC-134a which comprises reacting in the vapor phase at elevated temperature (300° to 400°C) a haloethane of formula CX₃CH₂Y, wherein X is Br Cl, or F and Y is Cl, with HF in the presence of a catalyst which is chromium oxide or which is at least in part basic chromium fluoride and wherein the HFC-134a product stream containing 1-chloro-2,2-difluoroethylene, which is an impurity, and HF is brought into contact over said catalyst at a temperature in the range of 100° to 275°C.

U.S. 4,258,225 discloses the reaction of trichloroethylene and HF over a TaF₅ catalyst to yield a mixture containing 1,2-dichloro-1,1-difluoroethane (41%), 1,1,2-trichloro-1-fluoroethane (57%), and 1,1,1,2-tetrachloroethane (2%).

GB 2,030,981 claims a process for the preparation of HFC-134a which comprises reacting HCFC-133a with HF in molar excess at a temperature not lower than 300°C in the presence of an inorganic chromium (III) compound with the introduction of 0.002 to 0.05 mol O₂ per mol of HCFC-133a into the reaction system. The patent also states that in this process, if the oxygen content is below the lower limit, catalyst deterioration occurs. When the oxygen content is more than the upper limit, catalyst deterioration is not a problem but the selective conversion to HFC-134a decreases. It is believed that this decrease in selectivity occurs because the catalyst promotes the oxidation of hydrogen chloride to molecular chlorine and water. [See Chemical Week, page 18, June 24, 1987 for the use of chromium based catalysts for the oxidation of hydrochloric acid to chlorine and water]. The highest yield of HFC-134a reported is 29%.

JP 55-27138 claims a process for the preparation of HFC-134a by the reaction of HCFC-133a and HF in the presence of an inorganic chromium (III) compound. The highest yield of HFC-134a reported is 35%.

U. S. 4,792,643 discloses a process for the manufacture of HFC-134a by the reaction of CX₂ =CHX, in which X is chlorine or bromine or a combination of both, with HF in the vapor phase at about 300°C to about 500°C over a catalyst prepared by codepositing hexavalent chromium oxide and a compound of a transition metal selected from the group consisting of titanium, zirconium, vanadium, molybdenum, and manganese on alumina, which is then contacted with HF, to form a product mixture from which HFC-134a is recovered. In one example 68% of trichloroethylene is converted to products with the following selectivities: 20% HFC-134a, 50% HCFC-133a and 30% other products, which include CClF=CHCl (FC-1121), CCl₂=CHF (FC-1121a), CF₃CHClF (HCFC-124), CHF₂CClF₂ (HCFC-124a) and CF₃CH₃ (HFC-143a). It is disclosed that HCFC-133a is available for further reaction either by extending the catalyst contact time, raising the temperature, or recycling.

The catalyzed reaction of HF with trichloroethylene to afford HFC-134a is a sequential reaction. The intermediate, HCFC-133a, can be produced in essentially quantitative yield; however the conversion in the second reaction of HCFC-133a with HF to yield final product, HFC-134a is dependent on the HF/HCFC-133a ratio, the higher the ratio, the more HFC-134a is produced at a given temperature. In general, HCFC-133a is isolated as an intermediate and fed to a second reactor where the conversion to HFC-134a is conducted. A need exists for the manufacture of HFC-134a from trichloroethylene and HF without simultaneously producing significant amounts of deleterious by-products or the requirement of two reactors, one for making HCFC-133a and one for making HFC-134a from HCFC-133a, which increases the cost of manufacture. This is particularly true in view of the growing demand for commercial quantities of HFC-134a as an environmentally desirable refrigerant. The herein described invention affords HFC-134a from the reaction of trichloroethylene and HF with very little (less than 5%) by-products in a single reaction zone.

### SUMMARY OF THE INVENTION

This invention provides an improved process for the manufacture of 1,1,1,2-tetrafluoroethane (HFC-134a) by the reaction of HF and trichloroethylene in the presence of 2-chloro-1,1,1-trifluoroethane (HCFC-133a) and a catalyst at elevated temperature to form a mixture comprising 1,1,1,2-tetrafluoroethane (HFC-134a), 2-chloro-1,1,1-trifluoroethane (HCFC-133a) and optionally other organic by-products. Said reaction is conducted in a single reaction zone at a temperature of about 300 to 500°C and at a contact time of 0.1 to 60 seconds and in the presence of a catalyst selected to form a mixture comprising 1,1,1,2-tetrafluoroethane and 2-chloro-1,1,1-trifluoroethane and less than 10 percent by weight of said other organic by-products; said catalyst being a catalyst composition comprising cobalt and trivalent chromium on an aluminium fluoride support. The 2-chloro-1,1,1-trifluoroethane (and, optionally, the said other organic by-products) in said mixture is recycled from the mixture to the reaction zone along with additional trichloroethylene in a molar amount at least equal to the molar amount of 1,1,1,2-tetrafluoroethane recovered from the mixture and with additional HF in a molar amount from 3 to 30 times the molar amount of trichloroethylene. The amount of trichloroethylene utilized in the recycle (2) in accordance with this invention is intended to include any unreacted trichloroethylehe which may be present in the mixture when HF is initially reacted with trichloroethylene.

In the practice of this invention only minor amounts, i.e. less than 10% by weight and more commonly less than 5% by weight, of other organic by-products, such as fluorinated ethylenes and other unsaturated haloolefins, are formed. Consequently, for all practical purposes the molar amount of trichloroethylene can be substantially at least equal to the molar amount of 1,1,1,2-tetrafluoroethane (HFC-134a) recovered. A key feature of the invention is that through control of recycle and, optionally, preferable catalyst selection, as described herein, the desired tetrafluoroethane can be obtained as the major product at greater than 99% trichloroethylene conversion with a reduction in the number of process steps for producing HFC-134a from trichloroethylene via HCFC-133a.

### DETAILS OF THE INVENTION

The catalyst composition utilized in the practice of this invention must contain at least cobalt and trivalent chromium on an aluminum fluoride support. By aluminum fluoride is meant at least one of AlF₃ and fluorided alumina. The AlF₃ and/or fluorided alumina can be prepared by any method known in the art or described hereinbelow. By fluorided alumina is meant a high fluorine content composition comprising aluminum, oxygen, and fluorine in such proportions that the total fluorine content or the catalyst composition taken as AlF₃ is, preferably, at least 50 weight percent, exclusive of any metal which is present, and more preferably 90 weight percent of the catalyst composition.

The total amount of metal, expressed as the metal, should be a catalytically effective amount and is generally less than 50% by weight of the catalyst composition and preferably less than 20% by weight of the catalyst composition, and usually at least 0.02% by weight of the catalyst composition. A more preferred range is 0.1 to 10% by weight of the Catalyst composition. The remainder of the catalyst composition may include alumina or aluminum oxyfluoride.

The form of the catalyst is not critical and may be used in the form of pellets, powders or granules.

In the practice of this invention the reaction of trichloroethylene with HF can be conducted in the presence or absence of oxygen.

The catalyst can be prepared in any manner known to the art. For example, the catalyst can be prepared by impregnating alumina or aluminum oxyfluoride with a solution of the metals which may be in the form of any soluble compound of the metal such as the oxide, oxyhalide, halide, pseudohalide, nitrate, sulfate, or organic salt such as acetate, propionate and any other compound of said metals which is convertible to a metal fluoride under the reaction conditions described herein. The halides include chlorides, fluorides, and bromides. The pseudohalides includes cyanides, cyanates and thiocyanates.

The catalyst can also be prepared by co-precipitation of the catalytic metals and the aluminum as the hydroxides which are thereafter dried and calcined to form the mixed oxides, a technique well known to the art. The resulting oxide can be fluorinated as described herein.

Generally, the catalyst composition preferred for use in the present invention will be pretreated with HF or other vaporizable compounds containing fluorine such as SiF₄, CCl₃F, CCl₂F₂, CHF₃ or CCl₂FCClF₂ to activate the catalyst. This pretreatment is accomplished by placing the catalyst composition in a suitable container which can be the reactor to be used to perform the reaction of the instant invention, and thereafter, passing HF over the dried catalyst composition. This is conveniently carried out by passing HF over the catalyst for a period of time, for example, of 15 to 300 minutes at a temperature of, for example, 200°C to 450°C. Nevertheless, this pretreatment is not essential; initial process conditions and equipment could be selected so as to activate the catalyst under initial process conditions.

By vaporizable fluorine-containing compound is meant a fluorine-containing compound which will convert the catalyst of the instant invention to the desired degree of fluorination using the pretreatment conditions described herein.

The reaction of trichloroethylene with HF in the presence of the catalyst of the instant invention is conducted at 300° to 500°C, preferably 350° to 425°C, and most preferably 370° to about 410°C, with a contact time of 0.1 to 60 seconds, preferably 5 to 30 seconds.

The amount of HF should be at least a stoichiometric amount. The molar ratio of additional HF to trichloroethylene recycled with HCFC-133a ranges from 3/1 to 30/1, preferably 5/1 to 15/1, and more preferably 5/1 to 10/1.

The amount of oxygen which may be present during the reaction relative to a mole of trichloroethylene and HCFC-133a can vary but will generally range from 0.001 to 1.0 moles. The oxygen may be fed to the reaction zone as such or may be diluted with an inert gas such as nitrogen, helium, or argon. The source of the oxygen may also be air containing molecular oxygen.

The catalyst preferred for use when oxygen is present in accordance with this invention also has the ability to minimize the oxidation of hydrogen chloride to molecular chlorine and water. The main disadvantage of this side reaction is that chlorine in the presence of HF reacts with CF₃CH₂F to produce CF₃CHFCl (HCFC-124) or it can react with CF₃CH₂Cl to produce CF₃CHCl₂ (HCFC-123). Both HCFC-123 and HCFC-124 can then further react with HF to produce CF₃CF₂H. This reaction with Cl₂ results in a significant yield loss of the desired product CF₃CH₂F. In addition the formed water in combination with HF is very corrosive.

In general, with a given catalyst composition, the higher the temperature, the greater the HF/trichloroethylene mol ratio, and the longer the contact time, the greater is the conversion to fluorinated products and the greater is the production of polyfluorinated products. Utilizing this invention, the above variables can be balanced, one against the other, so that formation of CF₃CH₂F is maximized and formation of the more highly fluorinated CF₃CHF₂ is minimized.

The reaction of trichloroethylene with HF may be conducted in any suitable reactor, including fixed and fluidized bed reactors. The reaction vessel should be constructed from materials which are resistant to the corrosive effects of hydrogen fluoride such as Inconel® alloy and Hastelloy® alloy.

Pressure is not critical. Atmospheric and superatmospheric pressures are the most convenient and are therefore preferred.

### EXAMPLES

In the following illustrative examples, all parts are by weight, all percentages are molar, and all temperatures are Celsius unless otherwise stated. All reactions used commercial HF containing only trace amounts of water.

### General Procedure for Fluorination

The reactor (a 0.5 inch ID, 12 inch long Inconel® alloy pipe) was charged with the amount of catalyst as described in the following examples, and placed in a sand bath. The bath was gradually heated to 400°C while N₂ gas at a flow rate of 50 cc/min was passed through the reactor to remove traces of water. The temperature was lowered to 200°C and HF and N₂ gas (1/4 molar ratio) were passed through the reactor and the N₂ flow was decreased with time until neat HF was being passed through the reactor. At this point the temperature was gradually raised to 425°C and maintained there for 15 to 300 minutes.

The temperature was then decreased to the indicated value and, thereafter, the other reactant flows were started. The flows were adjusted to give the indicated molar ratios and contact times in the Examples.

The reactor effluent was sampled on-line with a Hewlett Packard HP 5890 gas chromatograph using a 20 foot long, one-eighth inch diameter, column containing Krytox® perfluorinated polyether on an inert support and a helium flow of 35 cc/min. Gas chromatographic conditions were 70°C for three minutes followed by temperature programming to 180°C at a rate of 6°C/minute.

### (COMPARATIVE) EXAMPLE 1

### Fluorination of Trichloroethylene with HCFC-133a Recycle

The general procedure for fluorination was followed using 19.0 g (30 mL) of CoCl₂/Al₂O₃ (2% Co) as the initial catalyst charge. The HF/133a/trichloroethylene/O₂ molar ratio was 10/1/0.2/0.2. The product stream resulting from the reaction of HF with HCFC-133a, trichloroethylene and air over the prepared catalyst with a 20 second contact time at 390°C and after a reaction run of 53 hours showed the following: 17.4% CF₃CH₂F (HFC-134a), 80.8% CF₃CH₂Cl (HCFC-133a), 1.2% CF₂=CHCl (FC-1122), 0.3% C₂HCl₂F (FC-1121, two isomers), 0.1% trichloroethylene and 0.2% OH₂F₂.

The conversion of trichloroethylene was greater than 99% and the ultimate selectivity to HFC-134a with recycle of the HCFC-133a, FC-1122 and FC-1121 is greater than 98%.

### EXAMPLES 2-7

### Fluorination of Trichloroethylene with HCFC-133a Recycle

The general procedure for fluorination was followed using 21.1 g (30 mL) of (CoCl₂ + CrCl₃)/Al₂O₃ (1% Co + 1% Cr) as the initial catalyst charge. The results of the reaction of HF with either HCFC-133a and trichloroethylene, or HCFC-133a, trichloroethylene, and air over the prepared catalyst with a 20 second contact time are give in Table 1. The HF/133a/trichloroethylene/O₂ molar ratio was 10/1/0.2/0 for Examples 2-4 and 10/1/0.2/0.2 for Examples 5-7.

**TABLE 1**

| Ex. | Hrs | Temp | %CF₃CH₂F | %CF₃CH₂Cl | %CF₃CF₂H | %CFCl=CHCl |
|---|---|---|---|---|---|---|
| 2 | 2 | 380°C | 29.3 | 65.6 | 0.8 | 0.1 |
| 3 | 5 | 360 | 28.5 | 68.9 | 0.3 | 0.0 |
| 4 | 17 | 350 | 26.2 | 72.3 | 0.0 | 0.0 |
| 5 | 50 | 350 | 15.3 | 83.5 | 0.0 | 0.0 |
| 6 | 94 | 370 | 18.5 | 79.5 | 0.1 | 0.1 |
| 7 | 120 | 400 | 18.4 | 74.7 | 1.0 | 0.2 |

### Comparative EXAMPLES 8-14

### Fluorination of Trichloroethylene with HCFC-133a Recycle

The Inconel® reactor was charged with 31.0 g (30 mL) Cr₂O₃. The results of the reaction of HF with either HCFC-133a and oxygen, or HCFC-133a, trichloroethylene, and oxygen over the catalyst with a 20 second contact time are given in Table 2. The HF/133a/trichloroethylene/oxygen molar ratio was 10/1/0/0.2 for comparative examples 8 and 9 and 10/1/0.2/0.2 for 10-14. Comparison of these results with Examples 2-7 clearly show that the preferred catalyst embodiment of this invention provides many advantages, one of which is the fact that the chlorination chemistry which occurs with the catalyst of examples 8-14 affords significant amounts of by-products. Significant amounts of by-products are not formed when using the catalysts shown in Examples 2-7.

**TABLE 2**

| Ex. | Hrs | Temp | %CF₃CH₂F | %CF₃CH₂Cl | %CF₃CFHCl | %CF₃CF₂H | %CF₃CH₃ | %CHF₃ |
|---|---|---|---|---|---|---|---|---|
| 8 | 3 | 330°C | 24.0 | 68.7 | 2.6 | 1.9 | 0.0 | 1.0 |
| 9 | 5 | 350 | 23.2 | 62.5 | 3.3 | 7.2 | 0.0 | 1.7 |
| 10 | 17 | 350 | 15.7 | 72.6 | 3.5 | 5.0 | 0.0 | 0.9 |
| 11 | 23 | 370 | 12.2 | 72.6 | 1.5 | 10.9 | 0.3 | 0.9 |
| 12 | 26 | 390 | 12.5 | 68.3 | 0.6 | 13.8 | 1.5 | 1.5 |
| 13 | 29 | 410 | 10.8 | 57.2 | 1.2 | 22.2 | 3.1 | 2.5 |
| 14 | 31 | 430 | 7.4 | 39.5 | 2.4 | 36.9 | 5.0 | 4.2 |

## Claims

1. A process for the manufacture of 1,1,1,2-tetrafluoroethane by the reaction of HF and trichloroethylene in the presence of 2-chloro-1,1,1-trifluoroethane and a catalyst at elevated temperature to form a mixture comprising 1,1,1,2-tetrafluoroethane, 2-chloro-1,1,1-trifluoroethane and optionally other organic by-products, wherein
- the 2-chloro-1,1,1-trifluoroethane in said mixture is recycled from the mixture to the reaction zone along with additional trichloroethylene in a molar amount at least equal to the molar amount of 1,1,1,2-tetrafluoroethane recovered from the mixture and with additional HF in a molar amount from 3 to 30 times the molar amount of trichloroethylene, and
- said reaction is conducted in a single reaction zone at a temperature of 300 to 500°C and at a contact time of 0.1 to 60 seconds and in the presence of a catalyst selected to form a mixture comprising 1,1,1,2-tetrafluoroethane and 2-chloro-1,1,1-trifluoroethane and less than 10 percent by weight of said other organic by-products;
said catalyst being a catalyst composition comprising cobalt and trivalent chromium metal on an aluminum fluoride support.

2. The process of claim 1 wherein the reaction is conducted in the absence of oxygen.

3. The process of claim 1 wherein the amount of metal, expressed as the metal, in the catalyst composition is at least 0.02% by weight and less than 50% by weight of the catalyst composition.

4. The process of claim 1 wherein the aluminum fluoride is selected from at least one of AlF₃ and fluorided alumina.

5. The process of claim 4 wherein the fluorided alumina is a high fluorine-content composition comprising aluminum, oxygen, and fluorine in such proportions that the total fluorine content of the catalyst composition taken as AlF₃ is at least 50% by weight, exclusive of the metal, of the catalyst composition.

6. The process of claim 1 wherein the reaction is conducted in the presence of oxygen.

7. The process of claim 1 wherein the temperature is from 350°C to 425°C.

8. The process of claim 1 wherein the contact time is from 5 to 30 seconds.

9. The process of claim 1 wherein the additional HF is in a molar amount of 5 to 15 times the molar amount of trichloroethylene.

## Patentansprüche

1. Verfahren zur Herstellung von 1,1,1,2-Tetrafluorethan durch Umsetzung von HF und Trichlorethylen in Gegenwart von 2-Chlor-1,1,1-trifluorethan und eines Katalysators bei erhöhter Temperatur, um ein Gemisch zu bilden, das 1,1,1,2-Tetrafluorethan, 2-Chlor-1,1,1-trifluorethan und gegebenenfalls weitere organische Nebenprodukte umfaßt, bei dem
- das in dem Gemisch enthaltene 2-Chlor-1,1,1-trifluorethan aus dem Gemisch zusammen mit zusätzlichem Tri-Trichlorethylen in einer molaren Menge, die wenigstens der molaren Menge von 1,1,1,2-Tetrafluorethan ententspricht, die aus dem Gemisch gewonnen wurde, und mit zusätzlichen HF in einer molaren Menge, die der 3- bis 30fachen molaren Menge des Trichlorethylens entspricht, in die Reaktionszone recycelt wird, und
- die Reaktion in einer einzigen Reaktionszone bei einer Temperatur von 300 bis 500 °C und bei einer Kontaktzeit von 0,1 bis 60 sec und in Gegenwart eines Katalysators durchgeführt wird, der ausgewählt worden ist, um ein Gemisch zu bilden, das 1,1,1,2-Tetrafluorethan und 2-Chlor-1,1,1-trifluorethan und weniger als 10 Gew.-% der weiteren organischen Nebenprodukte umfaßt,
wobei der Katalysator eine Katalysatorzusammensetzung darstellt, die Cobalt und dreiwertiges Chrom auf einem Aluminiumfluorid-Träger umfaßt.

2. Verfahren nach Anspruch 1, bei dem die Reaktion in Abwesenheit von Sauerstoff durchgeführt wird.

3. Verfahren nach Anspruch 1, bei dem die Menge an Metall, ausgedrückt als Metall, in der Katalysatorzusammensetzung wenigstens 0,02 Gew.-% und Weniger als 50 Gew.-% der Katalysatorzusammensetzung beträgt.

4. Verfahren nach Anspruch 1, bei dem das Aluminiumfluorid ausgewählt wird aus wenigstens einem von AlF₃ und fluoriertem Aluminiumoxid.

5. Verfahren nach Anspruch 4, bei dem das fluorierte Aluminiumoxid eine Zusammensetzung mit hohem Fluorgehalt ist, umfassend Aluminium, Sauerstoff und Fluor in solchen Anteilen, daß der Gesamtfluorgehalt der Katalysatorzusammensetzung, herangezogen als AlF₃, ausschliesslich des Metalls, wenigstens 50 Gew.-% der Katalysatorzusammensetzung beträgt.

6. Verfahren nach Anspruch 1, bei dem die Reaktion in Gegenwart von Sauerstoff durchgeführt wird.

7. Verfahren nach Anspruch 1, bei dem die Temperatur 350 °C bis 425 °C beträgt.

8. Verfahren nach Anspruch 1, bei dem die Kontaktzeit 5 bis 30 sec beträgt.

9. Verfahren nach Anspruch 1, bei dem zusätzliches HF in einer molaren Menge des 5- bis 15fachen der molaren Menge von Trichlorethylen vorhanden ist.

## Revendications

1. Procédé pour la fabrication du 1,1,1,2-tétrafluoroéthane par la réaction de HF et de trichloréthylène en présence de 2-chloro-1,1,1-trifluoroéthane et d'un catalyseur à température élevée pour former un mélange comprenant du 1, 1, 1,2-tétrafluoroéthane, du 2-chlore-1,1,1-trifluoroéthane et, facultativement, d'autres sous-produits organiques, dans lequel
- le 2-chloro-1,1,1-trifluoroéthane dans ledit mélange est recyclé du mélange à la zone de réaction avec du trichloréthylène supplémentaire en une quantité molaire au moins égale à la quantité molaire du 1,1,1,2-tétrafluoroéthane récupéré du mélange, et avec du HF supplémentaire en une quantité molaire de 3 à 30 fois la quantité molaire du trichloréthylène, et
- ladite réaction est effectuée dans une seule zone de réaction à une température de 300 à 500 °C et avec une durée de contact de 0,1 à 60 secondes et en présence d'un catalyseur choisi pour former un mélange comprenant du 1,1,1,2-tétrafluoroéthane et du 2-chloro-1,1,1-trifluoroéthane et moins de 10 % en poids desdits autres sousproduits organiques ; ledit catalyseur étant une composition catalytique comprenant du cobalt et du chrome trivalent métalliques sur un support de fluorure d'aluminium.

2. Procédé selon la revendication 1, dans lequel la réaction est effectuée en l'absence d'oxygène.

3. Procédé selon la revendication 1, dans lequel la quantité de métal, exprimée en tant que métal, dans la composition catalytique est d'au moins 0,02 % en poids et inférieure à 50 % en poids de la composition catalytique.

4. Procédé selon la revendication 1, dans lequel le fluorure d'aluminium est choisi parmi au moins l'un parmi AlF₃ et l'alumine fluorée.

5. Procédé selon la revendication 4, dans lequel l'alumine fluorée est une composition à haute teneur en fluor comprenant de l'aluminium, de l'oxygène et du fluor en des proportions telles que la teneur totale en fluor de la composition catalytique prise sous la forme de AlF₃ Soit d'au moins 50 % en poids, à l'exclusion du métal, de la composition catalytique.

6. Procédé selon la revendication 1, dans lequel la réaction est effectuée en présence d'oxygène.

7. Procédé selon la revendication 1, dans lequel la température est de 350 °C à 425 °C.

8. Procédé selon la revendication 1, dans lequel la durée de contact est de 5 à 30 secondes.

9. Procédé selon la revendication 1, dans lequel le HF supplémentaire est dans une quantité molaire de 5 à 15 fois la quantité molaire du trichloréthylène.
